# EUROPÄISCHE PATENTANMELDUNG

(11) **EP 1 104 682 A1**
(43) Veröffentlichungstag der Anmeldung: **06.06.2001**
(21) Anmeldenummer: 99123520.1
(22) Anmeldetag: 01.12.1999
(51) Int. Cl.: A61M 1/16

(54) **Verfahren und Vorrichtung zum Austausch von Flüssigkeiten und/oder gelöster Substanzen insbesondere bei einer Dialysebehandlung**

(71) Anmelder: KFH Kuratorium für Dialyse und Nierentransplationen e.V., 63263 Neu-Isenburg (DE)
(72) Erfinder: Becker, Ferdinand, Dr., 63110 Rodgau (DE)
(74) Vertreter: Flosdorff, Jürgen, Dr.

(57) **Zusammenfassung**

Um das vor einer Dialysebehandlung festgesetzte Behandlungsziel eines vorbestimmten Volumenentzuges und einer vorbestimmten Reduzierung der Konzentration bestimmter gelöster Substanzen im Blut zu erreichen, wird durch Anordnung von Sensoren ein geschlossenes System gebildet, aus dem keine Flüssigkeit und keine gelöste Substanz entweichen kann, ohne vorher erfaßbar zu sein.

## Beschreibung

Die Erfindung betrifft ein Verfahren zum Austausch von Flüssigkeiten und/oder gelösten Substanzen durch eine Membran hindurch zwischen einem extrakorporalen Blutkreislauf und einem Kreislauf mit einer physiologischen Flüssigkeit, insbesondere bei einer Dialysebehandlung. Die Erfindung ist aber auch bei anderen Behandlungs- und Therapieverfahren wie z.B. bei einer Plasmaaphereseeinsetzbar.

Die Erfindung wird nachfolgend im Zusammenhang mit einer Dialysebehandlung beschrieben.

Wenn die natürlichen Funktionen der Niere nicht mehr ausreichen, um den Volumenentzug (Wasserentzug) und die Reduzierung harnpflichtiger Substanzen aus dem Blut sicherzustellen, muß bei dem Patienten eine Dialysebehandlung durchgeführt werden. Hierzu wird der Patient über einen extrakorporalen Blutkreislauf mit einem Dialysator verbunden, in dem ein Austausch von Flüssigkeit und gelösten Substanzen zwischen dem Blut einerseits und einer physiologischen Flüssigkeit andererseits durch eine Membran hindurch erfolgt. Dabei ist Ziel der Behandlung, nach Vorgabe einer Behandlungszeit das vor der Behandlung festgesetzte Behandlungsziel in Form eines vorbestimmten Volumenentzuges und einer vorbestimmten Reduzierung der Konzentration von bestimmten gelösten Substanzen im Blut zu erreichen.

Dieses Ziel wird mit den bisher bekannten Dialysegeräten nur ungenau erreicht.

Der vorliegenden Erfindung liegt die Aufgabe zugrunde, ein Verfahren und eine Vorrichtung anzugeben, mit dem bzw. der ein vor der Behandlung festgesetzter Flüssigkeitsentzug und eine vorgegebene Reduzierung der Konzentration bestimmter gelöster Substanzen im Blut genau erreichbar ist.

Diese Aufgabe wird erfindungsgmäß durch die Merkmale der Patentansprüche 1 und 13 gelöst.

Vorteilhafte Ausgestaltungen der Erfindung sind in den Unteransprüchen gekennzeichnet.

Bei dem erfindungsgemäßen Verfahren werden kontinuierlich oder in vorgegebenen kurzen Zeitintervallen von beispielsweise jeweils einer Minute Behandlungsdaten, insbesondere die Menge des Flüssigkeitsentzugs und/oder die Reduzierung der Konzentration bestimmter vorgegebener gelöster Substanzen und/oder die Größe der verbleibenden Konzentration dieser Substanzen im Blut erfaßt.

Die erfindungsgemäße Vorrichtung ist hierzu mit einer Sensoreinrichtung, die bevorzugt wenigstens zwei Sensoren umfaßt, versehen, die in der Lage sind, die vorgenannten Behandlungsdaten zu messen.

Hierdurch ist ein geschlossenes System geschaffen, aus dem keine Flüssigkeit oder gelöste Substanz entweichen kann, ohne genau erfaßbar zu sein.

Mit diesem System kann nicht nur der Wasserentzug und die Reduzierung der Konzentration bestimmter gelöster Substanzen im Blut genau gemessen werden, sondern es kann auch die Zuführung einer Flüssigkeit und/oder gelöster Substanzen aus einem Dialysierflüssigkeitskreislauf bzw. Kreislauf einer physiologischen Flüssigkeit durch eine Membran hindurch in den extrakorporalen Blutkreislauf erfaßt werden, so daß die Zuführung dieser Substanzen ins Blut exakt steuerbar ist.

Hierzu können in dem extrakorporalen Blutkreislauf in Strömungsrichtung vor und hinter der Membran bzw. dem Dialysator jeweils eine Sensoreinrichtung angeordnet sein, die mit den entsprechenden Meßeinrichtungen versehen ist. Zusätzlich oder statt dessen kann vorgesehen sein, daß in dem Dialysierflüssigkeitskreislauf ebenfalls vor und hinter dem Dialysator jeweils wenigstens eine Sensoreinrichtung angeordnet ist, die die Behandlungsdaten ermitteln kann, wobei die Anordnung so getroffen sein kann, daß den beiden Flußpumpen in dem Dialysierflüssigkeitskreislauf jeweils eine Sensoreinrichtung zugeordnet ist. Außerdem kann der Ultrafiltrationspumpe des Dialysierflüssigkeitskreislaufs eine Sensoreinrichtung zugeordnet sein.

In dem extrakorporalen Blutkreislauf können zwei Blutpumpen vorgesehen sein, denen jeweils eine Sensoreinrichtung zugeordnet ist.

Der Flüssigkeitsentzug des Blutes kann dabei in näherer Ausgestaltung der Erfindung dadurch gemessen werden, daß die Durchflußmenge des Blutes vor und hinter den Dialysator gemessen und die Differenz der Meßwerte gebildet wird. Alternativ hierzu oder zusätzlich kann die Durchflußmenge der physiologischen Flüssigkeit in dem Dialysierflüssigkeitskreislauf vor und hinter dem Dialysator bestimmt und die Differenz der Meßwerte gebildet werden. Sind beide Messungen erfolgt, kann der Flüssigkeitsentzug mit größter Genauigkeit ermittelt werden.

Die Reduzierung der Konzentration einer bestimmten gelösten Substanz kann ebenfalls dadurch gemessen werden, daß in einem oder in beiden Kreisläufen jeweils vor und hinter dem Dialysator die Konzentration gemessen und der Differenzwert bestimmt wird.

Bevorzugt ist jedoch, die im Blut verbliebene Konzentration der vorgegebenen gelösten Stoffe zu bestimmen, was zweckmäßigerweise durch eine in Fließrichtung vor dem Dialysator angeordnete Sensoreinrichtung im extrakorporalen Blutkreislauf erfolgt. Durch eine hinter dem Dialysator angeordnete Sensoreinrichtung kann - je nach Behandlungsart und Behandlungsziel- natürlich auch die Zuführung solcher Substanzen ins Blut genauestens gemessen werden.

Die ermittelten Daten werden einer Recheneinheit des Behandlungsgerätes, vorzugsweise eines Dialysegerätes oder eines externen Zusatzsystems, zugeführt, die mittels einer geeigneten Software diese Daten verarbeitet.

Aus dem kontinuierlich (oder in kurzen Zeitabständen) ermittelten Flüssigkeitsentzug läßt sich problemlos der seit Behandlungsbeginn erfolgte gesamte Flüssigkeitsentzug berechnen und mit dem festgesetzten Behandlungsziel vergleichen. Die jeweils erreichte Reduzierung der Konzentration der bestimmten gelösten Substanzen wird ebenfalls mit dem vorgegebenen Therapieziel verglichen.

Die gemessenen und entsprechend verarbeiteten Behandlungsdaten können analog und grafisch am Gerät dargestellt werden, wobei zusätzlich errechnet und angezeigt werden kann, wie lange die Behandlungszeit bei unveränderter Einstellung der Funktionselemente wie Pumpen etc. die Behandlung fortzusetzen ist. Die Recheneinrichtung des Gerätes kann ferner ermitteln, wie groß der gesamte Flüssigkeitsentzug und die jeweilige Reduzierung der Konzentration bestimmter gelöster Substanzen sein wird, bei Vorgabe einer noch verbleibenden Dialysezeit. Hieraus läßt sich errechnen, ob und gegebenenfalls wieviel Flüssigkeit und/oder bestimmte gelöste Substanzen dem extrakorporalen Blutkreislauf zugeführt werden müssen, um am Ende der Behandlung alle Behandlungsziele exakt zu ereichen.

Die ermittelten und errechneten Behandlungsdaten können der Steuer- bzw. Regeleinrichtung des Dialysekinetiksystems zugeführt werden, das diese Daten zur Steuerung bzw. Regelung von Funktionselementen des Gerätes verwendet. Beispielsweise können eine oder beide Blutpumpen oder Flußpumpen oder die Ultrafiltrationspumpe des Gerätes auf diese Weise gesteuert werden, wodurch beispielsweise eine Veränderung der zunächst vorgesehenen Behandlungszeit erfolgen kann.

Die ermittelten Daten können auch zur Veränderung der Zusammensetzung der physiologischen Flüssigkeit in dem Dialysierflüssigkeitskreislauf herangezogen werden, um beispielsweise die Reduzierung der Konzentration bestimmter gelöster Substanzen im Blut zu verlangsamen oder aber eine weitere Reduzierung der Konzentration zu verhindern.

Nach dem erfindungsgemäßen Verfahren und mit der erfindungsgemäßen Vorrichtung lassen sich die Behandlungsziele in Form eines vorgegebenen Volumenentzugs und einer vorgegebenen Reduzierung der Konzentration bestimmter gelöster Substanzen im Blut erreichen, da ein geschlossenes System geschaffen ist, bei dem exakt erfaßt wird, wieviel Flüssigkeit entwichen ist und in welchem Maße sich die Konzentration der bestimmten gelösten Stoffe reduziert hat.

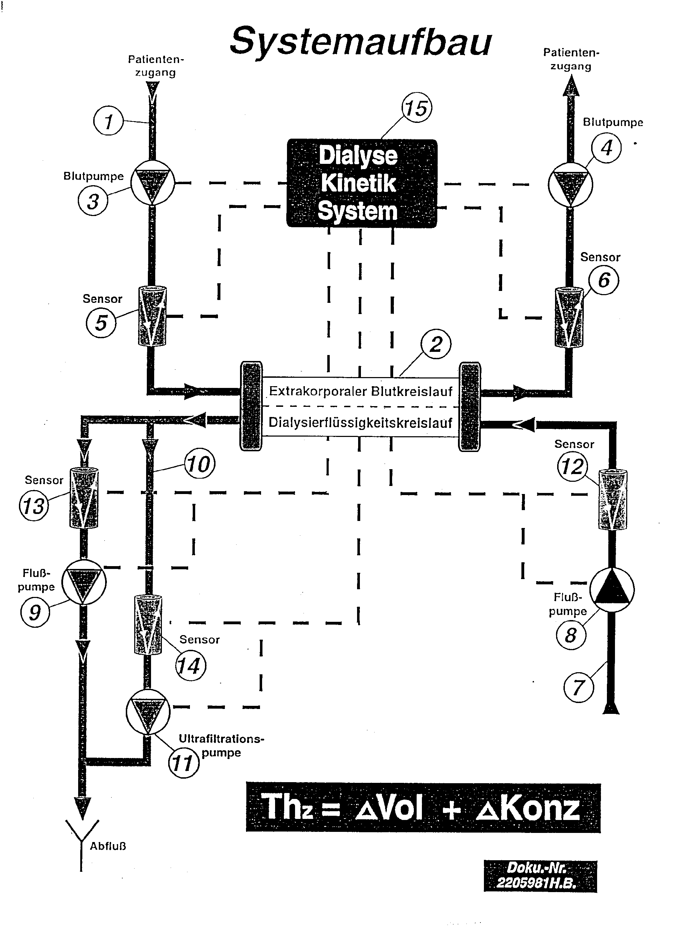

Nachfolgend wird anhand der beigefügten Zeichnung auf rein schematische Weise dieses System bei einem Dialysegerät beschrieben.

Der mit dem Blutsystem eines Patienten verbundene extrakorporale Blutkreislauf 1 enthält vor und hinter dem Dialysator 2 jeweils eine Blutpumpe 3, 4. Zwischen diesen beiden Blutpumpen und dem Dialysator 2 ist jeweils eine Sensoreinrichtung 5, 6 in den extrakorporalen Blutkreislauf eingeschaltet.

Der Dialysierflüssigkeitskreislauf 7 enthält auf ähnliche Weise zu beiden Seiten des Dialysators 2 jeweils eine Flußpumpe 8, 9, wobei an einer in Strömungsrichtung hinter dem Dialysator 2 liegenden Stelle eine Bypassleitung 10 abzweigt, in die eine Ultrafiltrationspumpe 11 eingeschaltet ist. In Strömungsrichtung hinter der Flußpumpe 8 sowie in Strömungsrichtung vor der Flußpumpe 9 und der Ultrafiltrationspumpe 11 sind jeweils Sensoreinrichtung 12, 13 und 14 angeordnet.

Die Sensoreinrichtungen 5, 6, 12, 13 und 14 sind in der Lage, mit geeigneten Meßeinrichtungen die Strömungsmenge und/oder die Konzentration bestimmter in den Flüssigkeiten gelöster Substanzen zu bestimmen. Die Strömungsmenge kann beispielsweise auch durch eine Meßeinrichtung an den Pumpen bestimmt werden.

Die kontinuierlich erfaßten Behandlungsdaten werden dem Dialysekinetiksystem 15 des Dialysegerätes zugeführt und in einem Rechner mittels einer geeigneten Software verarbeitet. Der jeweilige Ist-Zustand der Behandlung kann durch eine nicht dargestellte Anzeigeeinrichtung angezeigt werden. Durch Vergleich mit den vorgegebenen Behandlungszielen kann auch die verbleibende Behandlungszeit errechnet und angezeigt werden.

Die Erfindung sieht somit ein Verfahren mit folgenden Schritten vor: der Flüssigkeitsentzug und die Reduzierung der Konzentration vorgegebener gelöster Substanzen werden kontinuierlich oder in vorgegebenen kurzen Zeitintervallen erfaßt; die erfaßten Daten werden einer Rechen- und Steuereinheit des Gerätes zugeführt; die Recheneinheit vergleicht die ermittelten Werte mit den vorgegebenen Zielwerten und die Steuereinrichtung stellt die Funktionselemente des Gerätes automatisch so ein, daß am Ende der Behandlung die Zielwerte erreicht sind.

Die Recheneinrichtung kann dem kumulierten Flüssigkeitsentzug und die erreichte Reduzierung der Konzentration errechnen. Für den Fall der Beibehaltung der Einstellung der Funktionselemente kann die Recheneinrichtung die restliche Behandlungszeit ermitteln. Die Behandlungsdaten werden von Sensoreinrichtungen erfaßt.

## Patentansprüche

1. Verfahren zum Austausch von Flüssigkeiten und/oder gelösten Substanzen durch eine Membran zwischen einem extrakorporalen Blutkreislauf und einem Kreislauf mit einer physiologischen Flüssigkeit, insbesondere Dialysebehandlung,
**dadurch gekennzeichnet,**
daß Behandlungsdaten, insbesondere der Flüssigkeitsentzug und/ oder die Reduzierung der Konzentration vorgegebener gelöster Substanzen und/oder die verbleibende Größe der jeweiligen Konzentration dieser gelösten Substanzen erfaßt werden.

2. Verfahren nach Anspruch 1,
dadurch gekennzeichnet, daß die Behandlungsdaten kontinuierlich erfaßt werden.

3. Verfahren nach Anspruch 1,
dadurch gekennzeichnet, daß die Behandlungsdaten in vorgegebenen kurzen Zeitintervallen erfaßt werden.

4. Verfahren nach Anspruch 1,
dadurch gekennzeichnet, daß die Behandlungsdaten einer Rechen-und Steuereinheit des Gerätes zugeführt werden.

5. Verfahren nach einem der Ansprüche 1 bis 4,
dadurch gekennzeichnet, daß die Behandlungsdaten von wenigstens zwei Sensoreinrichtungen erfaßt werden, von denen eine in Strömungsrichtung vor der Membran und eine in Strömungsrichtung hinter der Membran angeordnet ist.

6. Verfahren nach Anspruch 5,
dadurch gekennzeichnet, daß die wenigstens zwei Sensoren in dem extrakorporalen Blutkreislauf angeordnet sind.

7. Verfahren nach Anspruch 5,
dadurch gekennzeichnet, daß die wenigstens zwei Sensoreneinrichtungen in dem Dialysierflüssigkeitskreislauf angeordnet sind.

8. Verfahren nach einem der Ansprüche 5 bis 7,
dadurch gekennzeichnet, daß fünf Sensoreinrichtungen angeordnet sind.

9. Verfahren nach einem der Ansprüche 1 bis 8,
dadurch gekennzeichnet, daß die gemessenen Daten einer Recheneinrichtung zugeführt werden, die den kumulierten Flüssigkeitsentzug und/oder die erreichte Reduzierung der Konzentration errechnet.

10. Verfahren nach einem der Ansprüche 1 bis 9,
dadurch gekennzeichnet, daß die Recheneinrichtung die ermittelten Werte mit den vorgegebenen Zielwerten vergleicht und für den Fall der Beibehaltung der Einstellung der Funktionselemente die restliche Behandlungszeit errechnet.

11. Verfahren nach einem der Ansprüche 1 bis 10,
dadurch gekennzeichnet, daß die jeweiligen Behandlungsdaten auf einer Anzeigeeinrichtung angezeigt werden.

12. Verfahren nach einem der Ansprüche 1 bis 11,
dadurch gekennzeichnet, daß die gemessenen und/oder errechneten Behandlungsdaten einer Steuer- oder Regeleinrichtung zur automatischen Einstellung der Funktionselemente des Gerätes zugeführt werden.

13. Vorrichtung zum Austausch von Flüssigkeiten und/oder gelösten Substanzen durch eine Membran zwischen einem extrakorporalen Blutkreislauf und einem Kreislauf mit einer physiologischen Flüssigkeit, insbesondere Dialysegerät,
gekennzeichnet durch wenigstens eine Sensoreinrichtung, mit der kontinuierlich der Flüssigkeitsentzug und/oder die Reduzierung der Konzentration vorgegebener gelöster Substanzen im Blut meßbar ist.

14. Vorrichtung nach Anspruch 13,
gekennzeichnet durch eine Rechen- und Steuereinrichtung, die die gemessenen Behandlungsdaten verarbeitet.

15. Vorrichtung nach Anspruch 13,
ferner gekennzeichnet durch eine Anzeigeeinrichtung für die jeweils gemessenen Behandlungsdaten.
